# EUROPÄISCHE PATENTANMELDUNG

(11) **EP 4 070 859 A1**
(43) Veröffentlichungstag der Anmeldung: **12.10.2022**
(21) Anmeldenummer: 21167559.0
(22) Anmeldetag: 09.04.2021
(51) Int. Cl.: A62B 18/02, A61L 9/20

(54) **ATEMSCHUTZMASKE**

(71) Anmelder: Herbst, Martin, 5090 Lofer (AT)
(72) Erfinder: Herbst, Martin, 5090 Lofer (AT)
(74) Vertreter: Grünecker Patent- und Rechtsanwälte PartG mbB

(57) **Zusammenfassung**

Die Erfindung betrifft eine Atemschutzmaske (1), umfassend ein Filtermaterial (11) aus einem luftdurchlässigen Material und wenigstens ein Befestigungsband (12), wobei das luftdurchlässige Material zumindest eine Lage eines Vliesstoffes (111) umfasst und das wenigstens eine Befestigungsband (12) dazu bestimmt ist, die Atemschutzmaske (1) am Kopf zu befestigen, dadurch gekennzeichnet, dass sie weiterhin eine Lichtquelle (16) zum Bereitstellen und Einbringen von erregerabtötendem Licht in das Filtermaterial (11) umfasst.

## Beschreibung

Die Erfindung betrifft eine Atemschutzmaske.

Atemschutzmasken bedecken regelmäßig Mund und Nase des Trägers mit einem Filtermaterial und dienen zu dessen Schutz vor in der Luft enthaltenen Schadstoffen und zum Schutz der Umgebung vor ausgeatmeten Bakterien und Viren. Insofern umfasst der Begriff unter anderem insbesondere einen Mund-Nasen-Schutz, medizinische Gesichtsmasken und filtrierende Halbmasken.

Hinsichtlich der typischen Formen unterscheiden sich "Medizinische Gesichtsmasken nach DIN EN 14683" und "Filtrierende Halbmasken nach DIN EN 149". Das verwendete Filtermaterial besteht heutzutage im Allgemeinen aus einem Vliesstoff, der aus Kunststoff hergestellt wird.

Während die medizinischen Gesichtsmasken eine meist rechteckige Form mit mehreren Falten aufweisen, sind die filtrierenden Halbmasken oft komplex dreidimensional geformt, um eine bessere Anpassung an das Gesicht zu ermöglichen. Dies kommt in der DIN EN 149 darin zum Ausdruck, dass Anforderungen hinsichtlich der Leckage der Maske zu erfüllen sind.

Eine geringe Leckage der Maske ermöglicht eine Erhöhung der Filterwirkung. Dabei basiert die Filterwirkung darauf, dass sich Luftschadstoffe und Krankheitserreger die von außen einwirken, oder ausgeatmet werden im Filtermaterial festsetzen und in diesem verbleiben. Durch das Verbleiben und Ansammeln der Luftschadstoffe und Krankheitserreger im Filtermaterial erhöht sich die Exposition des Trägers mit zunehmender Dauer des Tragens der Maske, wodurch die Verwendbarkeit der Atemschutzmaske begrenzt wird.

Viele Atemschutzmasken sind für den Einmalgebrauch gedacht und werden danach entsorgt. In bestimmten Fällen ist eine Reinigung der Maske, beispielsweise durch Waschen, oder Ausheizen möglich. Dies ist regelmäßig mit einer Unterbrechung der Verwendung und einem erhöhten Arbeitsaufwand für den Benutzer verbunden. Weiterhin wird die Lebensdauer der Atemschutzmaske durch wiederholte Reinigungsvorgänge begrenzt.

Chemische und biologische Schutzmasken nach Beispiel von US 2010/013271, die einen Filtereinsatz mit einem miniaturisierten, hochintensiven, kurzwelligen UV Desinfektionssystem vorsehen, bieten den Vorteil einer verlängerten Verwendbarkeit bei gleichzeitig geringer Exposition des Trägers, aber sind durch einen mitunter komplexen Aufbau gekennzeichnet.

Angesichts dessen besteht die der Erfindung zugrundeliegende Aufgabe darin, eine einfach herzustellende Atemschutzmaske mit guten Filter- bzw. Schutzeigenschaften und verlängerter Verwendbarkeit bereitzustellen.

Diese Aufgabe wird durch eine Atemschutzmaske gemäß Anspruch 1 gelöst.

Die Erfindung stellt eine Atemschutzmaske bereit, umfassend ein Filtermaterial aus einem luftdurchlässigen Material und wenigstens ein Befestigungsband, wobei das luftdurchlässige Material zumindest eine Lage eines Vliesstoffes umfasst und das wenigstens eine Befestigungsband dazu bestimmt ist, die Atemschutzmaske am Kopf zu befestigen, wobei die Atemschutzmaske weiterhin eine Lichtquelle zum Bereitstellen und Einbringen von erregerabtötendem Licht in das Filtermaterial umfasst.

Der Filterteil der Atemschutzmaske in Form des Filtermaterials ist dementsprechend aus einem luftdurchlässigen Material gebildet, das ein- oder mehrlagig aufgebaut sein kann. Das Filtermaterial stellt im Wesentlichen die Filterwirkung der Maske sicher.

Im Sinne der vorliegenden Erfindung bezeichnet ein "Vliesstoff" ein Wirrgelege, das einen Verfestigungsschritt (Vliesbindeschritt) durchlaufen hat, so dass es eine ausreichende Festigkeit aufweist, um insbesondere maschinell (also in industriellem Maßstab) zu Rollen auf- bzw. abgewickelt zu werden. Die für ein derartiges Aufwickeln minimal erforderliche Bahnspannung beträgt 0,044 N/mm. Die Bahnspannung sollte nicht höher als 10 % bis 25 % der Mindesthöchstzugkraft (gemäß DIN EN 29073-3:1992-08) des aufzuwickelnden Materials betragen. Daraus resultiert eine Mindesthöchstzugkraft für ein aufzuwickelndes Material von 8,8 N pro 5 cm Streifenbreite.

Der Begriff "Vliesstoff" ("Nonwoven") wird in anderen Worten gemäß der Definition nach ISO Standard ISO 9092:1988 bzw. CEN Standard EN 29092 verwendet. Details zur Verwendung der darin beschriebenen Definitionen und/oder Verfahren lassen sich auch dem Lehrbuch "Vliesstoffe", H. Fuchs, W. Albrecht, WILEY-VCH, 2012, entnehmen.

Die Atemschutzmaske kann eine filtrierende Halbmaske sein.

Die Atemschutzmaske kann insbesondere den FFP1 Standard gemäß DIN EN 149, den FFP2 Standard gemäß DIN EN 149, und/oder den FFP3 Standard gemäß DIN EN 149 erfüllen.

Die zumindest eine Lage eines Vliesstoffes kann durch ihre poröse Struktur dazu beitragen oder vorgesehen sein, die Erreger mechanisch aus der Luft und/oder einem Luftstrom zu filtern.

Die Bereitstellung von erregerabtötendem Licht kann beispielsweise durch eine oder mehrere lichtemittierende Dioden (LED) erfolgen.

Bei Erregern kann es sich beispielsweise um Viren, Bakterien, Aerosole, Feinstaub, oder Mikroorganismen handeln.

Die Bereitstellung und das Einbringen von erregerabtötendem Licht in das Filtermaterial gewährleistet eine verlängerte und sicherere Verwendbarkeit einer einfach herzustellenden Atemschutzmaske mit guten Filter- bzw. Schutzeigenschaften.

Bei dem erregerabtötenden Licht kann es sich um UV-C Strahlung handeln, wobei das Intensitätsmaximum der Lichtemission insbesondere zwischen 250 und 280 nm liegen kann.

Durch die Wahl der Strahlung und der Lage des Intensitätsmaximums um 265 nm ist gewährleistet, dass eine erbgutschädigende Wirkung insbesondere bei exponierten Viren und Bakterien erzielt wird. Dadurch wird eine Abtötung von Erregern und folglich eine Verringerung der Beladung der Atemschutzmaske mit aktiven Erregern erreicht. Dadurch kann die Atemschutzmaske länger sicher verwendet werden.

Bei der Atemschutzmaske kann das Filtermaterial den Maskenkörper bilden, der mit der Gesichtsform abschließt und die Atemschutzmaske kann einen formbaren Nasenbügel umfassen, der dazu dient, die Anpassung an die Gesichtsform zu optimieren.

Mit dem Maskenkörper kann der Teil der Atemschutzmaske gemeint sein, der die Gestalt, oder die äußere Erscheinung der Maske im Wesentlichen prägt. Mit Maskenkörper kann auch der Teil gemeint sein, der die Form, oder das Design der Maske wesentlich ausgestaltet.

Mit der Gesichtsform abschließend kann bedeuten, dass der Maskenkörper bei bestimmungsgemäßer Verwendung am Gesicht anliegt. Mit der Gesichtsform abschließend kann auch meinen, dass ein Umriss des Maskenkörpers bei bestimmungsgemäßer Verwendung mit dem Gesicht in Kontakt ist.

Mit einem formbaren Nasenbügel kann ein Element, Streifen oder Draht aus Metall, Kunststoff, oder anderem Material gemeint sein, das/der ein Mindestmaß an Verformbarkeit aufweist. Der formbare Nasenbügel kann mit dem Filtermaterial verklebt, vernäht, verschweißt, oder anderweitig an ihm befestigt sein. Überdies hinaus ist es möglich, dass der formbare Nasenbügel in einem umgrenzten Kanal verläuft, der in das Filtermaterial eingearbeitet ist, also nur unter Zerstörung der Atemschutzmaske von ihr lösbar ist.

Durch das Abschließen des Maskenkörpers mit der Gesichtsform und dem formbaren Nasenbügel wird eine Verringerung der Leckage der Atemschutzmaske erzielt. Dadurch wird die Filter- und Schutzeigenschaft der Atemschutzmaske erhöht.

Die Atemschutzmaske kann eine lichtleitende Lage zur Ausbreitung des erregerabtötenden Lichts über zumindest einen Teil der Fläche des Filtermaterials umfassen.

Die lichtleitende Lage kann die wenigstens eine Vliesstofflage des Filtermaterials der Atemschutzmaske selbst sein oder diese umfassen. Durch Einkopplung in die und diffuse Streuung an den Fasern des Vliesstoffs wird eine Ausbreitung des erregerabtötenden Lichts über zumindest einen Teil der Fläche des Filtermaterials erreicht. Mit Einkoppeln kann ein Einbringen gemeint sein, wobei unter Einkoppeln neben dem Durchstrahlen des Mediums alternativ oder zusätzlich auch die Ausbreitung im Medium zu verstehen ist. Dabei kann ein Teil der Lichtintensität von dem Filtermaterial absorbiert werden.

Die lichtleitende Lage kann auch als zusätzliche Lage des Filtermaterials realisiert sein. Dabei kann es sich bei der lichtleitenden Lage beispielsweise um Polymerfasern, oder ein Glasfasergelege handeln, das auf, unter, oder zwischen einer oder mehrerer weiterer Lagen des Filtermaterials zu liegen kommt. Das erregerabtötende Licht kann unter einem Winkel gegenüber der Faserrichtung gezielt in die lichtleitenden Fasern eingekoppelt werden, sodass entweder eine verstärkte Ausbreitung des Lichts entlang der Faserrichtung durch innere Totalreflexion an der Faser-Luft-Grenzfläche, oder vermehrt Transmission durch diese Grenzfläche auftritt. Dadurch kommt es zu einer gezielten Ausbreitung des erregerabtötenden Lichts über zumindest einen Teil der Fläche der Atemschutzmaske und das Einbringen desgleichen in das umliegende Filtermaterial.

In allen Fällen dient die lichtleitende Lage der gleichmäßigeren Ausbreitung des erregerabtötenden Lichts über zumindest einen Teil der Fläche der Atemschutzmaske und verbessert damit eine desinfizierende Wirkung im Filtermaterial, wodurch die sichere Verwendbarkeit der Atemschutzmaske verlängert wird.

Die Atemschutzmaske kann eine Steuereinheit zum Steuern eines oder mehrerer Betriebsparameter des erregerabtötenden Lichts umfassen.

Bei der Steuereinheit kann es sich um einen Prozessor, insbesondere einen Mikroprozessor, handeln, der wahlweise auf einen flüchtigen und/oder nichtflüchtigen Speicher zugreifen kann. Die Steuereinheit kann die Betriebsparameter des erregerabtötenden Lichts wie beispielsweise Dauer und Intensität der Strahlung, steuern oder regeln. Die Betriebsparameter können dabei einzeln einstellbar, oder in Form von einem oder mehreren Profilen auswählbar sein. Die Profile können in dem Speicher abgelegt sein. Beispielsweise ist ein Standardprofil, das eine mittlere Intensität bei konstanter Lichtabgabe vorsieht, denkbar neben Variationen wie einem hochintensiven Profil, das zur gründlichen Reinigung der Maske beiträgt, oder einem auf lange Betriebsdauer ausgelegten niedrigintensiven Profil. Weiterhin kann eine gepulste Lichtabgabe in wenigstens einem Profil vorgesehen sein.

In allen Fällen kann die Steuereinheit mit der Steuerung oder Regelung der Betriebsparameter des erregerabtötenden Lichts zur verlängerten sicheren Verwendbarkeit der Atemschutzmaske beitragen.

Das Wellenlängenspektrum des erregerabtötenden Lichts der Atemschutzmaske kann variabel einstellbar sein.

Durch Variation des Wellenlängenspektrums des erregerabtötenden Lichts, kann eine Anpassung der desinfizierenden Wirkung an bestimmte Erregergruppen ermöglicht werden. Weiterhin können energieökonomische Gründe bei der Wahl des Wellenlängenspektrums eine Rolle spielen. Außerdem kann es von Interesse sein, die Exposition beispielsweise des Trägers der Atemschutzmaske mit Licht einer bestimmten Wellenlänge zu vermeiden oder zu reduzieren. In diesem Fall ist es hilfreich, das Wellenlängenspektrum variieren zu können.

Die Variation des Wellenlängenspektrums des erregerabtötenden Lichts kann durch eine oder mehrere Multi-LED-Einheiten realisiert werden.

Mit Multi-LED-Einheit kann eine Einheit von mindestens zwei Einzel-LEDs gemeint sein. Die Multi-LED-Einheit kann insbesondere als baulich abgetrenntes Element ausgebildet sein.

Eine oder mehrere Einzel-LEDs einer Multi-LED-Einheit, eine Multi-LED-Einheit als Ganzes, oder eine Lichtquelle im Allgemeinen kann eine Optik zum Lichtleiten und gezielten Einkoppeln oder Einbringen des Lichtes in die lichtleitende Lage und/oder die wenigstens eine Vliesstofflage des Filtermaterials vorsehen. Die Optik kann in ihrer Wirkungsweise mit einer Linse vergleichbar sein. Beispielsweise kann die Wirkung der Optik darin bestehen, Lichtstrahlen zu bündeln, zu fokussieren, aufzuteilen, oder zu leiten etc. Die Optik kann erregerabtötendes Licht auf die Oberfläche des Filtermaterials richten. Hierzu kann die Optik reflektierende Elemente umfassen. Durch die Optik kann das erregerabtötende Licht alternativ oder zusätzlich seitlich in das Filtermaterial eingebracht werden.

Einzelne LEDs einer Multi-LED-Einheit können sich in ihrem individuellen Wellenlängenspektrum unterscheiden. Durch Wahl der individuellen Betriebsparameter einzelner LEDs einer Multi-LED-Einheit wie beispielsweise der Intensität können damit Variationen in dem resultierenden Wellenlängenspektrum aus überlagerten Einzel-Wellenlängenspektren realisiert werden.

Darüber hinaus kann die Variation des Wellenlängenspektrums einer einzelnen LED durch Variation der Temperatur möglich sein. In diesem Fall erfolgt die Wahl des Wellenlängenspektrums über die Einstellung der Temperatur. Die Einstellung der Temperatur kann beispielsweise durch Verwendung eines Peltier-Elementes ermöglicht werden. Das Peltier-Element kann zum Heizen, und/oder Kühlen, und/oder Halten einer bestimmten Temperatur einer oder mehrerer LEDs dienen.

In allen Fällen kann die Variation des Wellenlängenspektrums des erregerabtötenden Lichts den Anwendungsumfang der Atemschutzmaske erweitern und der verlängerten sicheren Verwendbarkeit der Atemschutzmaske dienen.

Die Atemschutzmaske kann eine Stromquelle zum Betrieb der erregerabtötenden Lichtquelle und/oder der Steuereinheit umfassen.

Bei der Stromquelle kann es sich um eine Gleichstromquelle handeln, insbesondere eine Batterie umfassend eine oder mehrere Primär- oder Sekundärzellen, beispielsweise eine 9V Blockbatterie, oder eine Kombination aus einer oder mehrerer solcher Batterien.

Die Spannung der Stromquelle kann beispielsweise durch eine elektronische Schaltung auf die zum Betrieb der erregerabtötenden Lichtquelle und/oder der Steuereinheit benötigte Spannung(en) angepasst werden.

Die Stromquelle kann an der Atemschutzmaske befestigt sein, beispielsweise seitlich im Bereich des Befestigungsbands oder des Maskenkörpers.

Die Stromquelle und/oder Steuereinheit und/oder Lichtquelle und/oder Optik und/oder lichtleitende Lage kann/können durch eine oder mehrere Befestigungseinheit(en) an dem Maskenkörper und/oder dem Befestigungsband lösbar befestigt sein. Dadurch kann eine Wiederverwendung insbesondere der Komponenten zur Lichterzeugung, -ausbreitung und - einbringung bei Austausch zumindest einer oder mehrerer Lagen des Filtermaterials erzielt werden. Die Befestigungseinheit(en) kann/können insbesondere als Klettverschluss und/oder Clip ausgestaltet sein. Es können insbesondere korrespondierende Befestigungseinheit(en) an dem Maskenkörper und/oder dem Befestigungsband und den jeweiligen zu befestigenden Elementen vorgesehen sein.

Insbesondere können Stromquelle, Steuereinheit, Lichtquelle und Optik eine bauliche Einheit bilden, die zerstörungsfrei lösbar an dem Maskenkörper und/oder dem Befestigungsband befestigt oder befestigbar ist.

Über den Betrieb der erregerabtötenden Lichtquelle und/oder der Steuereinheit dient die Stromquelle dem Zweck der verlängerten sicheren Verwendbarkeit der Atemschutzmaske.

Die Steuereinheit kann außerdem eine Schnittstelle umfassen, über welche ein externes Gerät auf die Steuereinheit zugreifen kann. Mittels einer solchen externen Schnittstelle können beispielsweise Änderungen an den Einstellungen der Atemschutzmaske vorgenommen werden.

Bei der externen Schnittstelle kann es sich um eine Kabel- oder Funkverbindung handeln. Insbesondere kann es sich um eine Universal Serial Bus (USB)-Schnittstelle, eine LAN-Schnittstelle, eine Bluetooth-Schnittstelle, eine WLAN-Schnittstelle, oder eine Infrarotschnittstelle handeln. Dadurch kann die Verbindung zu einem externen Gerät, beispielsweise einem Personal Computer (PC), einem Smartphone, einem MP3-Spieler, oder Vergleichbarem ermöglicht werden. Es kann eine volle Interaktivität der Atemschutzmaske mit dem externen Gerät erreicht werden. Einstellungen wie die Betriebsparameter oder die Wahl eines Profils können über das externe Gerät vorgenommen werden. Dies kann über eine Software oder Applikation auf dem externen Gerät, oder das Betriebssystem des externen Gerätes geschehen.

Die externe Schnittstelle dient zur Änderung der Einstellungen durch den Benutzer und kann damit zu einer verlängerten sicheren Verwendbarkeit der Atemschutzmaske beitragen.

Die Steuereinheit der Atemschutzmaske kann auf einer Software oder Firmware basieren und die Software oder Firmware kann über eine externe Schnittstelle aktualisiert werden.

Bei der externen Schnittstelle kann es sich um dieselbe externe Schnittstelle handeln, durch die Änderungen an den Einstellungen vorgenommen werden können. Es kann aber auch eine zweite, unterschiedliche oder gleiche Schnittstelle vorgesehen sein.

Durch die Aktualisierung der Software können beispielsweise geänderte und/oder neue Betriebsparameter und/oder Profile auf die Atemschutzmaske übertragen werden.

Dadurch trägt die Software zu einer verlängerten sicheren Verwendbarkeit der Atemschutzmaske bei.

Die vorliegende Erfindung wird anhand der nachfolgenden beispielhaften Ausführungen unter Bezugnahme auf die Figuren näher beleuchtet, ohne die Erfindung auf die speziellen dargestellten Ausführungen zu beschränken. Dabei zeigen
- Fig. 1: schematisch eine Atemschutzmaske,
- Fig. 2: schematisch eine Explosionsansicht einer Atemschutzmaske.

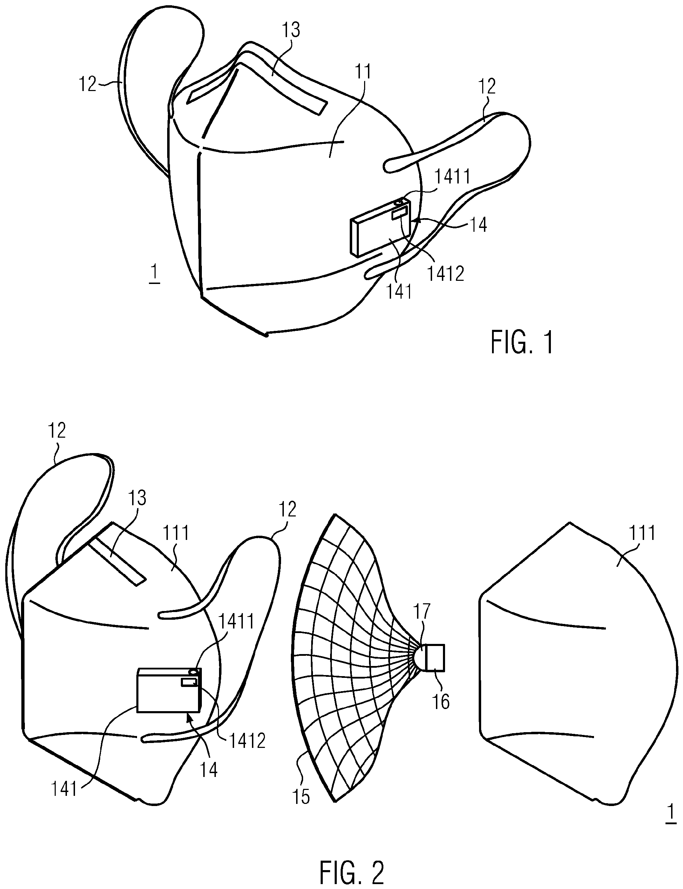

Fig. 1 illustriert den schematischen Aufbau eines Ausführungsbeispiels der Atemschutzmaske 1 in Form einer filtrierenden Halbmaske. Die dargestellte Atemschutzmaske 1 umfasst ein Filtermaterial 11, das eine dreidimensionale Struktur zur Anpassung an die Gesichtsform aufweist. Des Weiteren umfasst die gezeigte Atemschutzmaske 1 ein Paar Befestigungsbänder 12 zur Befestigung der Atemschutzmaske an den Ohren des Trägers. Die Endbereiche der Befestigungsbänder 12 sind mit dem Filtermaterial 11 verklebt, vernäht, verschweißt, oder anderweitig daran befestigt. Ein formbarer Nasenbügel 13 kann in das Filtermaterial 11 eingearbeitet sein und beispielsweise in einem Kanal fixiert sein, der durch Schweißnähte des Filtermaterials gebildet wird. Außerdem ist eine kombinierte Steuereinheit und Stromquelle 14 gezeigt, die in einem Gehäuse 141 seitlich an der Atemschutzmaske 1 angebracht ist. Das Gehäuse weist eine Öffnung für die externe Schnittstelle 1411 und einen ON/OFF-Schalter 1412 auf. Die externe Schnittstelle 1411 kann einen Verschluss zum Schutz der Kontakte vor eindringendem Wasser und Staub, oder anderen Verunreinigungen vorsehen. Der ON/OFF-Schalter 1412 dient dem Ein- und Ausschalten der elektrischen Funktionen der Maske.

Anhand der schematischen Explosionsansicht der Fig. 2 wird der innere Aufbau eines Ausführungsbeispiels der Atemschutzmaske 1 beschrieben. In der gezeigten Ausführung besteht das Filtermaterial 11 aus zwei Vliesstofflagen 111. Die Vliesstofflagen 111 können miteinander verklebt, vernäht, verschweißt, oder auf eine andere Art miteinander verbunden sein. Zwischen den beiden Vliesstofflagen 111 befindet sich eine lichtleitende Lage 15 umfassend ein Glasfasergelege. Mindestens eine Multi-LED-Einheit 16 koppelt mittels einer Optik 17 Licht in zumindest einen Teil der Glasfasern ein. Das Licht breitet sich entlang der Glasfasern aus und wird durch die räumliche Nähe zu den Vliesstofflagen in diese eingebracht. Die mindestens eine Multi-LED-Einheit 16 kann seitlich an dem Maskenkörper lösbar befestigt sein. Beispielsweise kann je eine Multi-LED-Einheit 16 pro Seite zur verbesserten Ausbreitung des erregerabtötenden Lichts über einen Teil der Fläche des Filtermaterials 11 lösbar befestigt sein. Die lösbare Befestigung kann beispielsweise durch einen Klettverschluss oder Clip verwirklicht sein.

Ein formbarer Nasenbügel 13 ist beispielsweise zwischen den beiden Vliesstofflagen 111 in einem Kanal, gebildet aus Schweißnähten mit den Vliesstofflagen, fixiert. Ein Paar Befestigungsbänder 12 ist beispielsweise von außen, also der dem Gesicht des Benutzers abgewandten Seite, mit den Vliesstofflagen verklebt, vernäht, verschweißt, oder anderweitig befestigt. Eine kombinierte Steuereinheit und Stromquelle 14 befindet sich in einem Gehäuse 141 seitlich an der äußeren Vliesstofflage 111 und speist über eine elektrische Verbindung die mindestens eine Multi-LED-Einheit 16. Das Gehäuse 141 weist eine Öffnung für die externe Schnittstelle 1411 und einen ON/OFF-Schalter 1412 auf. Die externe Schnittstelle 1411 kann einen Verschluss zum Schutz der Kontakte vor eindringendem Wasser und Staub, oder anderen Verunreinigungen vorsehen. Der ON/OFF-Schalter 1412 dient dem Ein- und Ausschalten der elektrischen Funktionen der Maske.

## Patentansprüche

1. Eine Atemschutzmaske (1) umfassend
ein Filtermaterial (11) aus einem luftdurchlässigen Material und
wenigstens ein Befestigungsband (12), wobei
das luftdurchlässige Material zumindest eine Lage eines Vliesstoffes (111) umfasst und
das wenigstens eine Befestigungsband (12) dazu bestimmt ist, die Atemschutzmaske (1) am Kopf zu befestigen,
**dadurch gekennzeichnet, dass** sie weiterhin eine Lichtquelle (16) zum Bereitstellen und Einbringen von erregerabtötendem Licht in das Filtermaterial (11) umfasst.

2. Die Atemschutzmaske (1) gemäß Anspruch 1, wobei es sich bei dem erregerabtötenden Licht um UV-C Strahlung handelt, wobei das Intensitätsmaximum insbesondere zwischen 250 und 280nm liegt.

3. Die Atemschutzmaske (1) gemäß einem der Ansprüche 1 bis 2, wobei
das Filtermaterial (11) den Maskenkörper bildet, der mit der Gesichtsform abschließt und
die Atemschutzmaske einen formbaren Nasenbügel (13) umfasst, der dazu dient, die Anpassung an die Gesichtsform zu optimieren.

4. Die Atemschutzmaske gemäß einem der Ansprüche 1 bis 3 umfassend eine lichtleitende Lage (15) zur Ausbreitung des erregerabtötenden Lichts über zumindest einen Teil der Fläche des Filtermaterials (11).

5. Die Atemschutzmaske (1) gemäß einem der Ansprüche 1 bis 4, weiterhin umfassend eine Steuereinheit (14), die die Betriebsparameter des erregerabtötenden Lichts steuert.

6. Die Atemschutzmaske (1) gemäß einem der Ansprüche 1 bis 5, wobei das Wellenlängenspektrum des erregerabtötenden Lichts variabel einstellbar ist.

7. Die Atemschutzmaske (1) gemäß einem der Ansprüche 1 bis 6, weiterhin umfassend eine Stromquelle (14) zum Betrieb der erregerabtötenden Lichtquelle und/oder der Steuereinheit (14).

8. Die Atemschutzmaske (1) gemäß einem der Ansprüche 5 bis 7, wobei mittels einer externen Schnittstelle (1411) Änderungen an den Einstellungen vorgenommen werden können.

9. Die Atemschutzmaske (1) gemäß einem der Ansprüche 5 bis 8, wobei die Steuereinheit (14) auf einer Software basiert und die Software mittels einer externen Schnittstelle (1411) aktualisiert werden kann.
